# EUROPEAN PATENT APPLICATION

(11) **EP 4 789 617 A1**
(43) Date of publication of application: **12.08.2026**
(21) Application number: 25156637.8
(22) Date of filing: 07.02.2025
(51) Int. Cl.: A61B 6/10, G21F 3/00

(54) **RADIATION-PROTECTIVE COVERING WITH FOLDABLE FLAP**

(71) Applicant: Texray AB, 412 92 Göteborg (SE)
(72) Inventor: APELL, Petra, 439 37 Onsala (SE); GELLERSTEDT, Fredrik, 413 09 Göteborg (SE)
(74) Representative: Öberg & Sjöberg AB

(57) **Abstract**

A radiation-protective covering (10) comprising: a flexible radiation-protective sheet (12) comprising a flexible radiation-protective material, the flexible radiation-protective sheet having a border (20), a radiation-protective flap (30) comprising a flexible radiation-protective material, the radiation-protective flap being attached to the flexible radiation-protective sheet (12) adjacent a part of the border (20), wherein the radiation-protective flap (30) is configured to be foldable from a first lowered position in which the radiation-protective flap is substantially parallel to the flexible radiation-protective sheet (12) to a second raised position. A use of the radiation-protective covering (10) for protecting clinical staff (8) from radiation (60) during a medical intervention comprising the use of radiation, is also disclosed.

## Description

### Technical field

The technology proposed herein relates to radiation-protective coverings, and particularly to X-ray protective coverings comprising a foldable flap.

### Background

Ionizing radiation, such as X-ray, is commonly used in radiologically guided interventions, such as cardiology, or Interventional Radiology (IR). The clinical staff is exposed to the radiation during the procedures. Current standards and practices are based on the premise that any radiation dose may result in unfavorable health effects and can result in the development of radiation-induced diseases such as a specific type of cancer, glioblastoma. Reducing the radiation exposure of the clinical staff is therefore important.

The radiation can be direct or scattered. In particular, radiation incident on a patient or a part of a patient will scatter, both at the point where the radiation impinges on the patient but also throughout the body of the patient. Further scattering occurs as the radiation impinges on the operating table.

During the radiologically guided intervention, or procedure, the patient is typically placed on an operating table. A source of radiation is provided above or underneath the operating table. The source of radiation may be positioned in various angular projections in relation to the patient. One or more radiation-protective drapes or coverings may be placed on the patient to prevent a major part of the radiation scattered in the body of the patient from irradiating out of the patient to reach the clinical staff. During the radiologically guided intervention the clinical staff typically stand along the left long side of the operating table for access to the interventional vascular entry points such as femoralis or radialis.

WO 2023/163643 discloses a radiation protection shielding system comprising a radiation protection drape sized to at least partially cover the patient from a proximity of a radiated area of the patient to the feet of the patient. As such, the radiation protection drape will prevent a major part of the radiation scattered in the body of the patient from irradiating out of the patient to reach the clinical staff.

There is a need for further reducing the extent of scattered radiation from a radiated area of the patent.

### Objects of the proposed technology

The proposed technology aims at providing an alternative to other radiation-protective drapes and coverings that is better suited to protect the clinical staff from radiation scattered from a radiated area of a patient.

It is a further object of the proposed technology to provide a radiation-protective covering that is easy to apply on the patient yet provides additional protection against scattered radiation from a radiated area of a patient.

### Summary

A first aspect of the proposed technology concerns a radiation-protective covering comprising:
- a flexible radiation-protective sheet comprising a flexible radiation-protective material, the flexible radiation-protective sheet having a border,
- a radiation-protective flap comprising a flexible radiation-protective material, the radiation-protective flap being attached to the flexible radiation-protective sheet adjacent a part of the border,
   ∘ wherein the radiation-protective flap is configured to be foldable from a first lowered position in which the radiation-protective flap is substantially parallel to the flexible radiation-protective sheet to a second raised position.

The present inventors have thus found that further protection from scattered radiation from an irradiated area of a patient, such as the area next to the radiation-protective sheet, or the area revealed through a fenestration or peripheral cutout as discussed further below, can be at least partially blocked from reaching the clinical staff by folding the radiation-protective flap to the second raised position so as to block at least part of the scattered radiation emanating from the irradiated area, i.e. from the area next to or beyond the border. The radiation-protective flap may for example block radiation scattered in angles of for example 15-45° to the horizontal plane of the patient and operating table. At the same time, the clinical staff can observe the irradiated area, i.e. area next to or beyond the border, such as the area of a cutout or fenestration, over the upper edge of the radiation-protective flap.

Further, when there is a need to interact with a part of the patient next to the border, e.g. such as an area revealed by a cutout or fenestration, such as during insertion or removal of a catheter or other medical instrument, the radiation-protective flap may be folded to the first lowered position so as to reduce or remove any interference by the radiation-protective flap on the interaction with the part of the patient. Expressed differently, the radiation-protective flap in its first lowered position provides little, or no, hindrance to accessing and interacting with a part of the patient that is revealed and/or accessible next to or beyond the border, such as through a cutout or fenestration. This ensures a good access and ergonomics for operating, i.e. interacting, with the patient. Before radiation is applied, the radiation-protective flap is raised, i.e. folded, to its second raised position in which it blocks at least part of the radiation emanating from, e.g. scattered from, the area next to or beyond the border.

The radiation-protective covering thus at least in part obviates the need for separate radiation-protective screens or shields being placed on or around the patient and/or operating table.

The radiation-protective covering may preferably be for covering at least a part of a patient during a clinical procedure involving radiation. Expressed differently, the radiation-protective covering has an intended position covering at least part of a patient, in particular a patient in a supine or prone position. The radiation-protective covering may be configured for at least partially covering a patient from the hip of the patient to the shoulder of the patient.

It is specified that the covering is radiation-protective. Expressed differently, the covering protects against, or attenuates, ionizing radiation. Expressed differently, the covering may be an X-ray protective covering. The covering may comprise a radiation attenuating material, in particular an X-ray attenuating material. The covering may be arranged or configured to attenuate X-ray radiation from a radiation source having a tube voltage of at least 40 kV, preferably at least 60 kV, and at the most 150 kV, preferably at the most 110 kV. For example, the covering may be arranged or configured to attenuate X-ray radiation from a radiation source having a tube voltage in the range of 40-150 kV, such as 60-110 kV. The covering may be arranged or configured to provide at least 0.05 mm, preferably at least 0.10 mm, more preferably at least 0.25 mm, most preferably at least 0.50 mm, and at the most 1.00 mm, preferably at the most 0.75 mm, more preferably at the most 0.50 mm Pb equivalence at any tube voltage in the range 60-110 kV. For example, the covering may be arranged or configured to provide 0.05 to 1.00 mm, preferably 0.10 to 1.00 mm, more preferably 0.25 to 1.00 mm, such as 0.25 to 0.75 mm or 0.30 to 0.60 mm, in particular at least 0.50 mm Pb equivalence, at any tube voltage in the range 60-110 kV. Pb equivalence is to be measured according to Inverse Broad Beam Geometry as described in IEC 61331-1:2014.

The covering may be non-transparent or non-translucent to optical, or visible, light.-

The flexible radiation-protective sheet is capable of flexing, or deforming, when subjected to force, such as force applied on the flexible radiation-protective material. The flexible radiation-protective material is thus pliable. The flexible radiation-protective material may be at least partially elastic, or resilient, whereby the flexible radiation-protective material essentially, or substantially, resumes an initial shape and configuration once a force deforming it is no longer applied.

The flexible radiation-protective sheet may preferably be generally rectangular. Expressed differently, the flexible radiation-protective sheet may have opposite upper and lower edges, and opposite left and right sides. The upper edge of the flexible radiation-protective sheet is generally positioned closer to the head of a patient when the radiation-protective covering is in the intended position than the lower edge, which generally is positioned closer the feet of the patient when the radiation-protective covering is in the intended position. In connection with a peripheral cutout, as described further below, generally rectangular encompasses that the flexible radiation-protective sheet, in the absence of the peripheral cutout, could be considered to form a rectangle.

The radiation-protective covering, and in particular the flexible radiation-protective sheet, may generally have a length, from the lower edge to the upper edge of the flexible radiation-protective sheet, of at least 20 cm, preferably at least 40 cm, and at the most 100 cm, preferably at the most 80 cm, such as 20 to 100 cm, preferably 40 to 80 cm, and a width, from the left edge to the right edge of the flexible radiation-protective sheet, of at least 20 cm, preferably at least 40 cm, and at most 100 cm, preferably at the most 80 cm, such as 20 to 100 cm, preferably 40 to 80 cm.

The flexible radiation-protective sheet may further have a thickness of at least 1 mm, preferably at least 2 mm, and at the most 10 mm, preferably at the most 5 mm, such as 1 to 10 mm, preferably 1 to 5 mm or 2 to 5 mm.

The flexible radiation-protective sheet may alternatively have other shapes such as an L-shape or rectangular hexagon, a polygonal shape such as a pentagonal shape or an octagonal shape, a circular or elliptical shape, or a trapezoidal shape. For such shapes, the length of the radiation-protective covering, and in particular the flexible radiation-protective sheet, extends between the point on the edge of the flexible radiation-protective sheet that is closest to the head of a patient when the radiation-protective covering is in the intended position, and the opposite point on the edge that is positioned closest to the feet of the patient when the radiation-protective covering is in the intended position. Further, the width extends between the points on the edge of the radiation-protective covering that are furthest from each other along a line perpendicular to the length.

The flexible radiation-protective material is flexible as described above for the flexible radiation-protective sheet. The flexible radiation-protective material may comprise, or consist of, a permeable fabric, or fibrous material. The permeable fabric may comprise filaments. The filaments may be arranged in a regular pattern. The filaments may extend in a uniform direction. Each filament may be of a solid composition that comprises a carrier substance and a radiopaque substance. For example, the solid composition may be a polymeric composite material. The filaments may be arranged in a regular pattern. It is understood that each filament may be a monofilament. It is further understood that the carrier substance and the radiopaque substance may be mixed, and that the radiopaque substance may be evenly distributed within the carrier substance. The carrier substance may comprise at least one polymer and the radiopaque substance may comprise at least one metal. For example, the polymer may be a thermoplastic polymer such a polyvinyl chloride or a polyolefin, and the metals may, as a powder, be as an element, oxide or alloy of for example Lead, Barium, Antimony, Bismuth, or Tungsten (Wolfram), and any combination thereof.

The fabric may be a woven fabric with the filaments forming the weft of the fabric. It is understood that the fabric has a warp, for example of polyester. More specifically, the first material may be any of the radiation-protective material described in WO2014163574A1. The fabric contributes to higher longevity of the radiation-protective covering.

Alternatively, the flexible radiation-protective material may be, or comprise, an impermeable sheet. The impermeable sheet may be of a solid composition that comprises a carrier substance and a radiopaque substance. For example, the solid composition may be a polymeric composite material. It is further understood that the carrier substance and the radiopaque substance may be mixed, and that the radiopaque substance may be evenly distributed within the carrier substance. The carrier substance may comprise at least one polymer and the radiopaque substance may comprise at least one metal.

It is understood that the flexible radiation-protective material may be composed of several layers, for example two layers of fabric or two impermeable sheets. As specified above for the radiation-protective covering, the flexible radiation-protective material may have at least 0.05, mm, preferably at least 0.10 mm, more preferably at least 0.25 mm, most preferably at least 0.50 mm, and at the most 1.00 mm, preferably at the most 0.75 mm, more preferably at the most 0.50 mm Pb equivalence at any tube voltage in the range 60-110 kV. For example, the covering may be arranged or configured to provide 0.05 to 1.00 mm, preferably 0.10 to 1.00 mm, more preferably 0.25 to 1.00 mm, such as 0.25 to 0.75 mm or 0.30 to 0.60 mm, most preferably at least 0.50 mm Pb equivalence at any tube voltage in the range 60-110 kV.

The flexible radiation-protective material may have a thickness of at least 0,5 mm, preferably at least 1 mm. The flexible radiation-protective material may have a thickness of at the most 10 mm, preferably at the most 5 mm, such as 0.5 to 10 mm, preferably 1 to 5 mm or 2 to 5 mm.

It is specified that the flexible radiation-protective sheet has a border. Expressed differently, the flexible radiation-protective sheet has an edge, i.e. a peripheral edge, which delimits the flexible radiation-protective sheet. Further expressed differently, the border or edge of the flexible radiation-protective sheet is made up of the circumference, e.g. the sides and corners of the radiation-protective sheet.

If the flexible radiation-protective sheet comprises a fenestration, then the circumference border, or edge of the fenestration is also a border of the flexible radiation-protective sheet. Further, if the flexible radiation-protective sheet comprises a peripheral cutout, then the circumference, border or edge of the peripheral cutout is also a border of the flexible radiation-protective sheet.

It is specified that the radiation-protective flap is attached to the flexible radiation-protective sheet adjacent at least a part of the border. The part of the border is not the full length of the border. The part of the border may for example be a part of a side of the flexible radiation-protective sheet, a part of a circumference or border of a fenestration, or a part of a circumference, border or edge of a peripheral cutout.

The part of the border is preferably at the most 50% of the length of the border, more preferably at the most 30% of the length of the border, most preferably at the most 20% of the length of the border.

The radiation-protective flap is radiation-protective as described above for the radiation-protective covering.

The radiation-protective flap preferably has a length which is larger than its width, i.e. height. The length and width of the radiation-protective is preferably determined with the radiation-protective flap in the first lowered position. The length of the radiation-protective flap is in particular determined as the extension of the radiation-protective flap along the part of the border adjacent which the radiation-protective flap is attached. The width of the radiation-radiation-protective flap is thus perpendicular to the length of the radiation-protective flap.

The radiation-protective flap may preferably have a height of at least 5 cm, more preferably at least 10 cm. The width of the radiation-protective flap may preferably be at the most 40 cm, more preferably at the most 30 cm, most preferably at the most 20 cm, so as to not impede visibility of the area beyond the border, for example the area of a fenestration or peripheral cutout. The length of the radiation-protective flap may preferably be at least 10 cm, more preferably at least 20 cm. The length of the radiation-protective flap is preferably not more than 60 cm, more preferably not more than 50 cm, most preferably not more than 40 cm.

The flexible radiation-protective material of the radiation-protective flap is radiation-protective as described above for the radiation-protective covering, and is further preferably the same type of flexible radiation-protective material as described for the flexible radiation-protective sheet.

The radiation-protective flap is attached to the flexible radiation-protective sheet. The attachment is preferably permanent, such as by stitching, adhesive, welding or laminating, the radiation-protective flap to the flexible radiation-protective sheet or by forming the radiation-protective flap integrally with the flexible radiation-protective sheet. Alternatively, the attachment is releasable, such as by hook and loop attachment, magnetic attachment, snap fasteners, or zippered attachment of the radiation-protective flap to the flexible radiation-protective sheet.

It is specified that the radiation-protective flap is attached to the flexible radiation-protective sheet adjacent at least a part of a circumference of the at least one fenestration or at least one peripheral cutout. Expressed differently, the radiation-protective flap is attached to the flexible radiation-protective sheet such that it in the second raised position is configured to block or attenuate at least a part of radiation emanating from the peripheral cutout or fenestration. The radiation-protective flap may be attached to the flexible radiation-protective sheet at the part of the border
Accordingly, adjacent is not limited to the radiation-protective flap being attached right up to, or along, the part of the border. Rather, the radiation-protective flap may be attached to the flexible radiation-protective sheet spaced apart from the part of the border by a distance measured perpendicular to the border and perpendicularly to the line along which the radiation-protective flap is attached to the flexible radiation-protective sheet. The distance is preferably at the most the height of the radiation-protective flap, more preferably at the most 2/3 of the height of the radiation-protective flap, most preferably at the most 1/3 of the height of the radiation-protective flap. Generally, the closer the radiation-protective flap is attached to the part of the border, i.e. the lower the distance, the more of radiation emanating from beyond the part of the border can be blocked. Preferably the distance is the same, or less, than the width of the radiation-protective flap. More preferably the distance is less than the width of the radiation-protective flap as this provides for using the radiation-protective flap to cover at least part of the area beyond the part of the border, such as least part of a fenestration or peripheral cutout.

The flap may be attached directly at, i.e. along, the part of the border, edge or circumference. The distance is however preferably at least 1 or 2 cm, more preferably at least 5 cm, as this decreases the risk of leakage of radiation between the flap and the sheet, e.g. through the attachment, or through any wear on the radiation-protective flap due to it being folded repeatedly from the first lowered position to the second raised position and vice versa.

It is specified that the radiation-protective flap is configured to be foldable. Expressed differently, the radiation-protective flap is capable of folding from a compact folded state, i.e. the first lowered position, to a more extensive or larger unfolded state, i.e. the second raised position.

In the first lowered position the radiation-protective flap is substantially parallel to the flexible radiation-protective sheet. Expressed differently, the first lowered position is one in which the radiation-protective flap may contact the flexible radiation-protective sheet. The angle between the radiation-protective flap and the flexible radiation-protective sheet in the first lowered position may for example be at the most 15°, preferably at the most 10°, more preferably at the most 5°, most preferably 0°. Accordingly, the term substantially parallel encompasses that the angle between the radiation-protective flap and the flexible radiation-protective sheet may be 0° to 15°, preferably 0° to 10°, more preferably 0° to 5°, most preferably 0°.

The first lowered position preferably has the upper edge of the radiation-protective flap, i.e. the edge of the flap opposite to the edge along which the radiation-protective flap is attached to the flexible radiation-protective sheet, positioned furthest from the part of the border.

The second raised position is different from the first lowered position. Expressed differently, the angle between the radiation-protective flap and the flexible radiation-protective sheet in the second raised position is larger than the angle between the radiation-protective flap and the flexible radiation-protective sheet in the first lowered position. The angle between the radiation-protective flap and the flexible radiation-protective sheet in the second raised position may for example be at least 60°, preferably at least 75°, more preferably at least 85°, most preferably at least 90°. The angle between the radiation-protective flap and the flexible radiation-protective sheet in the second raised position may for example be at the most 120°, preferably at the most 105°, more preferably at the most 95°, most preferably 90°. The angle between the radiation-protective flap and the flexible radiation-protective sheet in the second raised position may thus be from 60° to 120°, preferably 75° to 105°, more preferably 85° to 95°, most preferably 90°.

The radiation-protective flap may be substantially perpendicular to the flexible radiation-protective sheet in the second raised position.

The radiation-protective flap may be raised to the second raised position manually. The radiation-protective flap may further be retained in the second raised position manually, i.e. by holding it.

Preferably the radiation-protective flap is attached to the flexible radiation-protective sheet along one edge of the radiation-protective flap such that a fold line may be defined in the flexible radiation-protective material of the radiation-protective flap along and adjacent the edge.

The width of the radiation-protective flap and the distance between the attachment of the radiation-protective flap and the part of the border, and the dimensions of the area beyond the border, may be configured such that the radiation-protective flap covers at least part of, preferably all of, the area beyond the part of the border in the third position the first lowered position. Alternatively, the radiation-protective flap may further be configured to the be foldable to a third position 180° from the first lowered position. In either case, if the part of the border is a part of the border of a fenestration, then the radiation-protective flap may cover part or all of the fenestration in the first lowered position and the third position, third position respectively. This may be used to prevent or attenuate radiation emanating from the area beyond the border, e.g. from the area of the fenestration when the radiation-protective flap is in the first lowered position or the third position, respectively, and to provide access to the fenestration and provide at least partial protection from radiation emanating from the fenestration by moving the radiation-protective flap to the second raised position.

Preferably, the flexible radiation-protective sheet comprises at least one fenestration or peripheral cutout, and the part of the border is a part of a border of the at least one fenestration or peripheral cutout.

This is especially advantageous since peripheral cutouts and fenestration may be used to provide access to the patient, and as providing the radiation-protective flap adjacent part of the border of such fenestrations and peripheral cutouts increases radiation protection when using the peripheral cutout or fenestration.

The term fenestration refers to an opening, or aperture, or hole, in the flexible radiation-protective sheet. The border, or circumference, of the fenestration is thus made up by the flexible radiation-protective sheet around the fenestration. The fenestration may for example be circular, quadrilateral, such as square, elliptical, or oblong. The fenestration may have a diameter, or largest dimension, of for example at least 1 cm, preferably at least 2 cm, and at the most 40 cm, preferably at the most 20 cm, such as 1 to 40 cm, preferably 2 to 20 cm, more preferably 2 to 15 cm.

The term peripheral cutout refers to a cutout formed in the periphery, i.e. along the border, of the flexible radiation-protective sheet. Expressed differently, a peripheral cutout is formed by removing material from the periphery, or border, of the flexible radiation-protective sheet. A peripheral cutout, in contrast to a fenestration, is thus not surrounded by the flexible radiation-protective sheet on all sides. A peripheral cutout is rather surrounded by the radiation-protective sheet on two sides, such as when the peripheral cutout is formed by removing material at a corner of the radiation-protective sheet, or surrounded by the flexible radiation-protective sheet on three sides, such as when the peripheral cutout is formed by removing material at a position on one side, or border, of the radiation-protective sheet. An opening, or aperture, or hole, in the flexible radiation-protective sheet formed by providing a slit extending between a fenestration and a side or border of the flexible radiation-protective sheet is considered a fenestration.

The peripheral cutout may for example be quadrilateral, such as square or rectangular. The peripheral cutout may have a diameter, or largest dimension, of for example at least 1 cm, preferably at least 2 cm, such as 1 to 40 cm, preferably 2 to 20 cm, more preferably 2 to 15 cm. The diameter, or largest dimension, of the peripheral cutout may preferably be at the most 40 cm, preferably at the most 20 cm.

When determining the dimensions of a peripheral cutout the cutout is considered to be delimited by the periphery, or border, of the flexible radiation-protective sheet in the absence of the peripheral cutout. Thus, for a cutout formed at a corner of the flexible radiation-protective sheet, the intersection of lines drawn parallel to the borders, or sides, of the flexible radiation-protective sheet adjacent the corner delimit the cutout. Similarly, for a peripheral cutout formed by removing material at a position on one side, or border, of the radiation-protective sheet, a line drawn along that one side, or border, delimits the peripheral cutout.

Preferably the flexible radiation-protective sheet comprises more than one fenestration or peripheral cutout.

Preferably the flexible radiation-protective sheet comprises a peripheral cutout or fenestration configured to provide access to the torso of the patient when the radiation-protective covering is in the intended position covering a patient.

Preferably the flexible radiation-protective sheet comprises a peripheral cutout or fenestration configured to provide access to the hip and/or groin of the patient when the radiation-protective covering is in the intended position covering a patient.

Preferably the flexible radiation-protective sheet comprises a peripheral cutout or fenestration configured to provide access to the shoulder of the patient when the radiation-protective covering is in the intended position covering a patient.

Preferably the radiation-protective covering further comprises:
- a retaining element for retaining the radiation-protective flap in the second raised position.
This is advantageous in that is helps ensure that the radiation-protective flap remains in the second raised position, thus obviating the need for manually holding the radiation-protective flap in the second raised position, as well as decreasing the risk that the radiation-protective flap resumes the first lowered position which would entail that the radiation-protective flap would not protect against radiation emanating from beyond or next to the part of the border.

The retaining element may for example comprise a shaping element, e.g. a plastically deformable element, such as a sheet, ribbon or wire extending between the radiation-protective flap and the flexible radiation-protective sheet for supporting the radiation-protective flap in the second raised position.

Alternatively, the retaining element may comprise an elongated element attached to the flexible radiation-protective sheet and being releasably attachable to the radiation-protective flap in the second raised position. Further alternatively, the radiation-protective flap may be provided with a bendable, curvable or foldable portion, allowing that portion of the radiation-protective flap to be bent, curved or folded away from or towards the part of the border, such as a part of the border of a peripheral cutout or fenestration to support the radiation-protective flap against the flexible radiation-protective sheet.

Preferably the radiation-protective flap has a length in the plane of the flexible radiation-protective sheet in the first lowered position, and the radiation-protective flap is attached to the flexible radiation-protective sheet along a part of its length.

This is advantageous in that it allows the part of the radiation-protective flap that is not attached to the flexible radiation-protective sheet to be curved or bent, e.g. to provide a retaining element as described above and/or to better conform the shape of the radiation-protective flap to that part of the border, such as to a curvature of the part of the border adjacent to which the radiation-protective flap is attached.

50% to 100%, preferably 50% to 80%, more preferably 50 to 70%, of the length the radiation-protective flap is attached to the flexible radiation-protective sheet. Accordingly, up to 50% of the length of the radiation-protective flap may not be attached to the flexible radiation-protective sheet.

Regardless of how much of the length of the radiation-protective flap that is attached to the flexible radiation-protective sheet, it is preferred that the radiation-protective flap is attached to the flexible radiation-protective sheet along a straight attachment line to facilitate folding. An attachment line is a line extending along the attachment of the radiation-protective flap to the flexible radiation-protective sheet.

However, it is also possible to attach the radiation-protective flap to the flexible radiation-protective sheet along a curved or bent attachment line. This makes folding more difficult as the radiation-protective flap and/or the flexible radiation-protective sheet may need to deform, and thereby resist folding, for the radiation-protective flap to be folded between the first lowered position and the second raised position, and vice versa. However, the resistance to folding, and in particular the curved attachment line, may be used as a retaining element retaining the radiation-protective flap in the second raised position by resisting folding thereof to the first lowered position.

Preferably the radiation-protective flap comprises a shaping element for retaining a shape of the radiation-protective flap in the second raised position, preferably wherein the shaping element is arranged along a longitudinal edge of the radiation-protective flap, more preferably wherein the shaping element is arranged along a first longitudinal edge opposite to a second longitudinal edge along which the radiation-protective flap is attached to the flexible radiation-protective sheet.

This is advantageous in that it may serve as a retaining element for retaining the radiation-protective flap in the second raised position, as well as serving to maintain a desired shape of the radiation-protective flap in the second raised position.

The shaping element may alternatively be referred to as a support element because it supports a shape of the radiation-protective flap.
a longitudinal edge of the radiation-protective flap is an edge along the length of the radiation-protective flap. The first longitudinal edge may be considered an upper edge of the radiation-protective flap, whereas the second longitudinal edge may be considered a lower edge of the of the radiation-protective flap.

The shaping element is attached to, or fixed to, the radiation-protective flap. The shaping element is deformable by hand. Worded differently, the shaping element may be configured to deform plastically at a load that can be applied by hand. It is understood that this is without any tools. It is understood that the load may localized, or more specifically at a load point. It is further understood that the load can be applied to the shaping element as such, and/or via the radiation-protective flap.

It is understood that the shaping element may be configured to elastically deform at a first load on the shaping element and plastically deform at a second load on the shaping element. It is further understood that the second load is greater than the first load, or more specifically that the second load is caused by forces in the same direction but greater than the forces that causes the first load. It is further understood that the shaping element has a yield point between the first load and the second load.

The shaping element may be of metal. It is understood that the metal may be an alloy, such as steel or nitinol. Alternatively, the shaping element may be of plastic, such as high-density polyethylene. It is understood that the plastic may a reinforced plastic, such as glass-fibre reinforced polyethylene.

The shaping element is preferably plastically deformable so as to be able to maintain the shape to which it formed.

The shaping element may be elongated. It may have a first end and an opposite second end. It is understood that the element extends between the first end and the second end. It is further understood that the shaping element may be curved or bent in an intended curvature of the radiation-protective flap as conforming to the circumference of the peripheral cutout or fenestration.

The shaping element may comprise, or be, a wire, band, strip of sheet. The shaping element may be solid. For example, it may be a solid-core wire. Alternatively, the shaping element may be hollow. For example, it may be a tube. The shaping element may have a circular or square cross section. For example, it may be a wire with a circular or square cross section. Alternatively, the shaping element may have a flattened, or rectangular, cross section, such as a band or a strip.

Preferably the radiation-protective flap comprises:
∘ a first flap portion attached to the flexible radiation-protective sheet adjacent a first part of the part of the border, and
∘ a second flap portion attached to the flexible radiation-protective sheet adjacent a second part, different from the first part, of part of the border,
∘ and wherein the first and second flap portions overlap at least partially in the second raised position.

This is advantageous in that it provides for better conforming the shape of the radiation-protective flap to the part of the border, such as to a curvature of the part of the border.

The first flap portion and the second flap portion may preferably have the same dimensions. The first flap portion and the second flap portion are separate from each other. The first flap portion and the second flap portion may preferably be foldable from the first lowered position to the second raised position independently of each other. The first flap portion and the second flap portion may however overlap at least partially in the first lowered position, in which case one of the first flap portion and the second flap portion may not be foldable independently of the other.

It is specified that the second flap portion is attached to the flexible radiation-protective sheet adjacent a second part, or second section, different from the first part, or first section, of the part of the border. Expressed differently, the first and second flap portions are not attached to the same attachment line on the flexible radiation-protective sheet, nor are the first and second flap portions attached adjacent the same part of the part of the border. The first part and the second part, while different, may overlap partially. For a border, e.g. border of a peripheral cutout or fenestration, having a first curved part and a second curved part, the first and second flap portions may preferably be attached to the flexible radiation-protective sheet along first and second attachment lines aligned tangentially with the respective first and second curved parts. As such, the first and second attachment lines will generally not be parallel but form an angle between them in the plane of the flexible radiation-protective sheet.

Preferably, as described above for the radiation-protective flap, only a part of the length of the respective first and second flap portions are attached to the flexible radiation-protective sheet.

Preferably the first and second flap portions are attached to the flexible radiation-protective sheet along corresponding first and second intersecting attachment lines in the plane of the flexible radiation-protective sheets, and
the retaining element comprises attachment elements provided on the first and second flap portions for connecting the first and second flap portions to each other in the second raised position.

This is advantageous in that the attachment elements represent a retaining element for retaining the radiation-protective flap in the second raised position. Specifically, as the first and second intersecting attachment lines, due to the intersection, will form an angle in the plane of the flexible radiation-protective sheet, any further folding of the first or second flap portions will result in a movement of the first and second flap portions relative to each other. Such movement is however prevented by the attachment elements interconnecting the first and second flap portions, and the radiation-protective flap is thereby retained in the second raised position. It is specified that the first and second attachment lines are intersecting. Expressed differently, a line extending along the attachment of the first flap portion to the flexible radiation-protective sheet should eventually intersect a line extending along the attachment of the second flap portion to the flexible radiation-protective sheet. Accordingly, it is not required that the attachment of the flap portions per se, e.g. the lines of stitching or adhesive, etc, physically intersect.

Preferably the first and second flap portions overlap with parts of the first and second flap portions that are not attached to the flexible radiation-protective sheet.

This allows those parts to curve or bend to better conform to the part of the border, such as a curvature of the part of the border.

Preferably the attachment elements are provided on parts of the first and second flap portions which overlap with each other.

This ensure allows the attachment elements to be provided between the parts of the first and second flap portions that overlap in the second raised position such that the attachment elements are not subjected to radiation.

Preferably the attachment elements comprise one or more selected from the group consisting of hook and loop structures, magnets, snap fasteners, button and buttonhole, hook and eye, toggle and loop, twist lock, and buckles.

These types of attachment elements provide readily attachable, ad detachable, attachment of the first and second flap portions to each other.

Preferably the radiation-protective flap is attached to the flexible radiation-protective sheet by one or more of stitches, hook and loop, magnets, rivets, snap fasteners, welding, and adhesive.

These methods of attaching the radiation-protective flap are easily implemented.

Preferably flexible radiation-protective sheet comprises at least one fenestration or peripheral cutout, and the radiation-protective covering further comprises:
- a radiation-protective patch comprising a flexible radiation-protective material, the radiation-protective patch being configured to be attachable to the flexible radiation-protective sheet for at least partially covering the at least one fenestration or peripheral cutout in the flexible radiation-protective sheet.

This is advantageous in that it allows further covering of peripheral cutouts or fenestrations which are not needed for a certain clinical procedure. This further provides an alternative to using the radiation-protective flap in its first position, or in the third position, to cover at least part of the fenestration or peripheral cutout.

The flexible radiation-protective material may be the same as described above for the flexible radiation-protective sheet.

The radiation-protective patch may be configured to be attachable to the flexible radiation-protective sheet by use of one or more of hook and loop structures, magnets, snap fasteners, button and buttonhole, hook and eye, toggle and loop, twist lock, and buckles, provided on the flexible radiation-protective sheet and on the radiation-protective patch.

Preferably the radiation-protective covering comprises at least two of at least one fenestration or at least one peripheral cutout, and further comprises at least two radiation-protective flaps.

This is advantageous in that it provides radiation-protection, by the radiation-protective flaps, at each of the at least two of the at least one fenestration or at least one peripheral cutout.

Each of the radiation-protective flaps may be as described above.

Preferably the flexible radiation-protective material comprises:
a permeable fabric that comprises filaments, each filament being of a solid composition that comprises a carrier substance and a radiopaque substance, and the fabric is a woven fabric with the filaments forming the weft of the fabric, or
an impermeable sheet formed from a solid composition comprising a carrier substance and a radiopaque substance.

These are suitable flexible radiation-protective materials and have been described in further detail above.

Additionally, when the filaments extend in a uniform direction, the uniform direction may be aligned with, or parallel with, the length of the radiation-protective flap. This makes the flexible radiation-protective material easier to fold and increases longevity of the radiation-protective flap
Alternatively, the uniform direction may be aligned with, or parallel with, the height of the radiation-protective flap, this increases stiffness of the radiation-protective flap and prevents sagging or dropping of the upper edge of the radiation-protective flap when the height of the radiation-protective flap is large.

For the flexible radiation-protective sheet the uniform direction is preferably aligned with the length of the radiation-protective covering to better conform the covering to the shape of the patient.

Preferably the flexible radiation-protective material comprises a permeable fabric that comprises filaments as described above. Such a permeable fabric has high tolerance, i.e. is more durable, to being bent, or deformed, when the radiation-protective flap is folded.

A second aspect of the proposed technology relates to a use of the radiation-protective covering according to the first aspect of the proposed technology for protecting clinical staff from radiation during a medical intervention comprising the use of radiation.

The medical intervention may comprise a radiologically guided intervention, such as cardiology, or Interventional Radiology (IR). The medical intervention, or medical procedure, may comprise diagnostic and/or surgical procedures. The medical intervention may be a fluoroscopic, or fluoroscopy, assisted intervention.

The use may comprise covering at least a part of a patient with the radiation-protective covering.

The use may further comprise folding the radiation-protective flap from the first lowered position to the second raised position.

The use may alternatively comprise folding the first and second flap portions from the first lowered position to the second raised position and attaching the first and second flap portions to each other using the attachment elements.

The clinical staff may comprise any personnel present during the medical intervention.

The third aspect of the proposed technology may alternatively be considered a method of protecting clinical staff from radiation during a medical intervention comprising the use of radiation, comprising the steps of:
- providing:
   - the radiation-protective covering according to the first aspect of the proposed technology,
- covering at least a part of a patient with the radiation-protective covering, and
- folding the radiation-protective flap from the first lowered position to the second raised position.

### Brief description of the drawings

A more complete understanding of the abovementioned and other features and advantages of the technology proposed herein will be apparent from the following detailed description of preferred embodiments in conjunction with the appended drawings, wherein:
**Fig. 1a** shows a first embodiment of a radiation-protective covering with a radiation-protective flap, comprising first and second flap portions, in the first lowered position.
**Fig. 1b** shows the first embodiment of the radiation-protective covering with the first flap portion folded to the second raised position.
**Fig. 1c** shows the first embodiment of the radiation-protective covering with the first flap portion curved to conform to the peripheral cutout in the flexible radiation-protective sheet.
**Fig. 1d** shows the first embodiment of the radiation-protective covering with the second flap portion folded to the second raised position.
**Fig. 1e** shows the first embodiment of the radiation-protective covering with the second flap portion curved to conform to the first flap portion and attached to the first flap portion.
**Fig. 1f** shows a second embodiment of the radiation-protective covering further comprising a radiation-protective patch attached to the flexible radiation-protective sheet.
**Fig. 1g** shows a third embodiment of the radiation-protective covering comprising two radiation-protective flaps, each comprising first and second flap portions.
**Fig. 1h** shows a fourth embodiment of the radiation-protective covering in which the radiation-protective flap, in particular the first and second flap portions, is provided with a shaping element, and in which one of the peripheral cutouts in the flexible radiation-protective sheet is replaced with a fenestration.
**Fig. 1i** shows an enlarged partial cross section of the fourth embodiment of the radiation-protective covering showing the placement of the shaping element within and along an outer edge of the radiation-protective flap, in particular within and along an outer edge of the first flap portion.
**Fig. 2** shows a patient laying on an operating table and being partially covered by the radiation-protective covering, and further shows a C-arm imaging device and a clinical staff member.

### Detailed description

In the figures and the below description, the same reference numerals are used for the same or related features. One or more ' added to a reference number indicates that the feature so designated is a variant of the feature bearing the reference number without the '. A subscript number indicates that the features so references is a further one of the feature bearing the reference number without the subscript number.

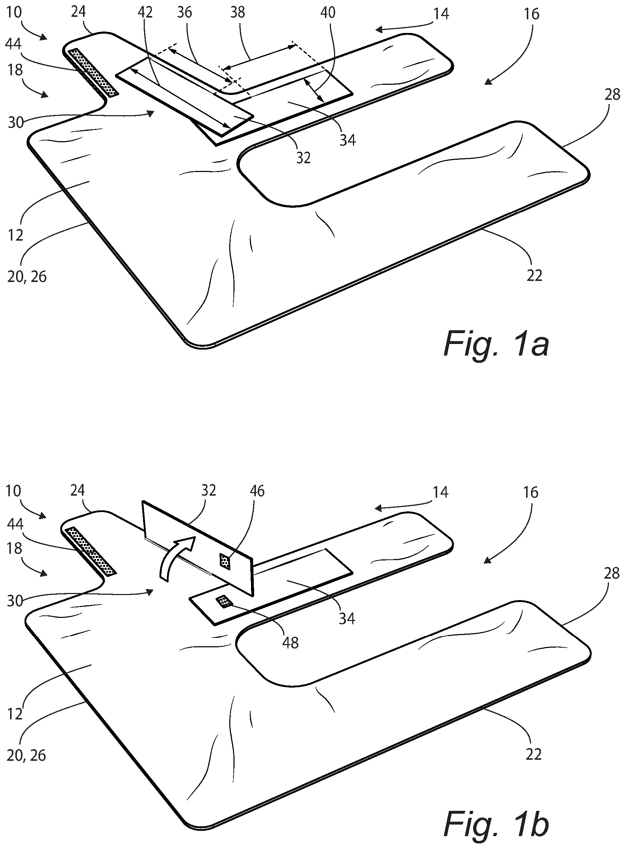

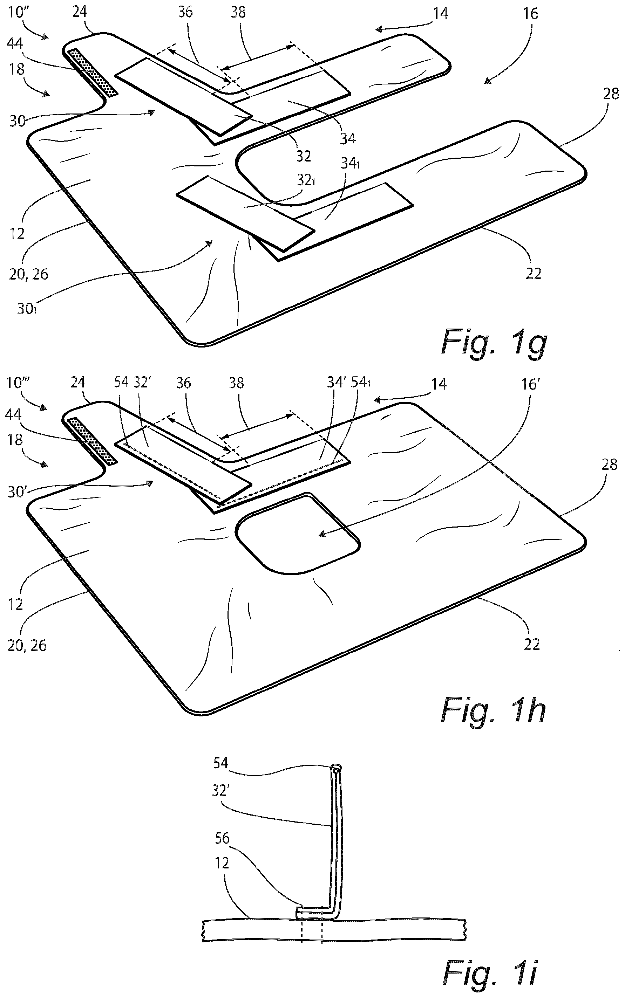

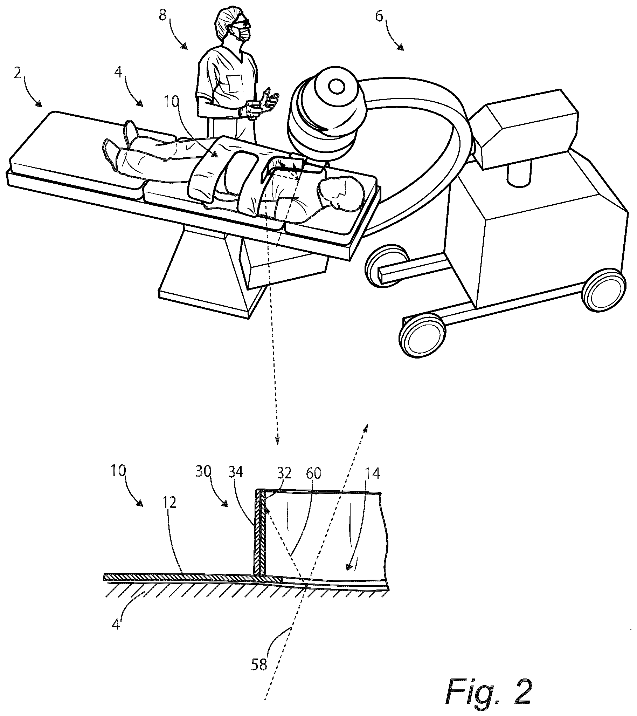

**Fig. 1a** shows a first embodiment of a radiation-protective covering 10 comprising a flexible radiation-protective sheet 12 comprising a flexible radiation-protective material and having a generally rectangular shape and comprising first, second and third peripheral cutouts 14, 16, 18. As further shown in Fig. 2, the first peripheral cutout 14 is configured to provide access to the torso of a patient, the second peripheral cutout 16 is configured to provide access to a hip of the patient, and the third peripheral cutout is configured to provide access to a shoulder of the patient. The first, second and third peripheral cutouts are formed in the periphery, border or circumference 20 of the flexible radiation-protective sheet 12 which otherwise, absent the peripheral cutouts as can be understood from Fig. 1a, would have a rectangular shape. As seen in Fig. 1h, one or more of the first, second and third peripheral cutouts 14, 16, 18 may be replaced by a fenestration, and/or further peripheral cutouts and/or fenestrations may be provided in the flexible radiation-protective sheet 12.

As can be seen in Fig. 1a the borders of the respective first, second and third fenestrations 14, 16, 18 are part of the border 20 of the flexible radiation-protective sheet 20.

The flexible radiation-protective sheet 12 further comprises a lower edge, or border, 22, an upper edge, or border, 24, a left edge, or border, 26 and a right edge, or border, 28.

As seen, the first peripheral cutout 14 is formed at the corner between the upper edge 24 and the right edge 28. Lines drawn along the upper edge 24 and the right edge 28 thus delimits the first peripheral cutout 14 when determining the dimensions of the first peripheral cutout 14.

The second peripheral cutout 16 is formed in the right edge 28. A line drawn along the right edge 28 thus delimits the second peripheral cutout 16 when determining the dimensions of the second peripheral cutout 16.

The third peripheral cutout 18 is formed at the corner between the first edge 26 and the upper edge 24. Lines drawn along the upper edge 24 and the left edge 26 thus delimit the third peripheral cutout 18 when determining the dimensions of the third peripheral cutout 18.

The radiation-protective covering 10 further comprises a radiation-protective flap 30 comprising a flexible radiation-protective material. The radiation-protective flap 30 is shown comprising first and second flap portions 32, 34. Each of the first and second flap portions 32, 34 are attached to the flexible radiation-protective sheet 12 along a part of its lengths as indicated by arrows 36, 38 also indicating first and second intersecting attachment lines. As seen, the first and second flap portions 32, 34, and thus the radiation-protective flap 30, is attached to the flexible radiation-protective sheet 12 adjacent, e.g. along but slightly apart from, such as 1-5 cm from, a part of the border 20 of the flexible radiation-protective sheet 20, in particular adjacent a part of the border, or circumference, of the first peripheral cutout 14. Implementing the radiation-protective flap 30 as first and second flap portions 32, 24 is advantageous in that it allows the radiation-protective flap 30 to better conform to any curvature in the part of the border adjacent to which the first and second flap portions 32, 24 are attached, such as any curvature in the border or circumference of the peripheral cutout or fenestration. However, it is also possible to implement the radiation-protective flap 30 as a single flap portion, e.g. as the first flap portion 32 only.

As seen, the first and second flap portions 32, 34, and thus the radiation-protective flap 30, may have a width, indicated by arrow 40 of at least 5 cm, such as 10 cm as shown in Fig. 1a. As will be seen, the width may alternatively be considered a height of the flap in the second raised position. Further, each of the first and second flap portions may have a length, as indicated by arrow 42 of 40 cm. As seen, the first and second flaps portions 32, 34 overlap over a length of about 10 cm when in the first lowered position as shown in Fig. 1a.

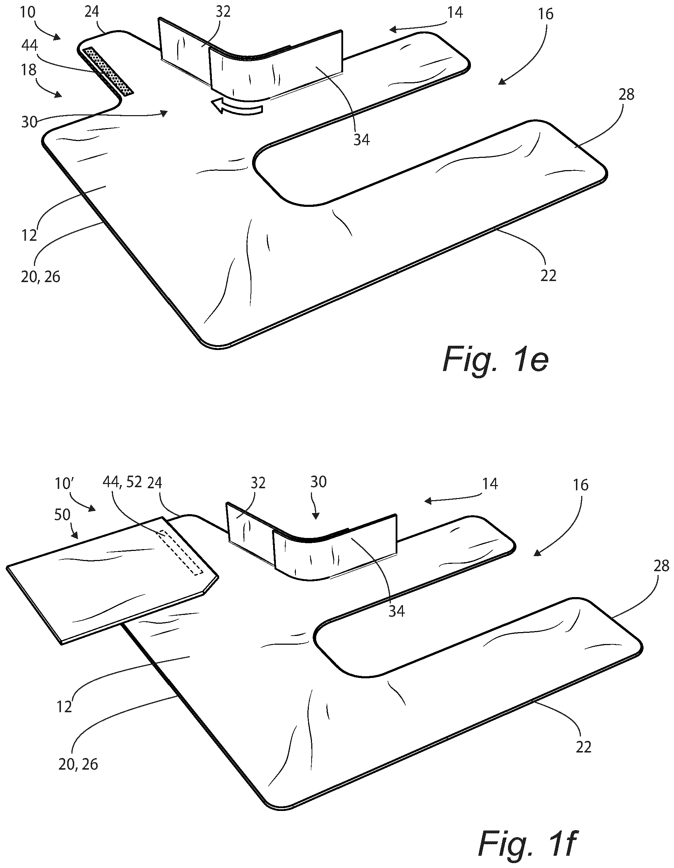

Further shown in Fig. 1a is an attachment element in the form of a hook and loop attachment strip 44, the function of which will be further discussed in relation to Fig. 1f.

As noted above, Fig. 1a shows the first and second flap portions 32, 34 in the first lowered position in which they are substantially parallel, or lies flat onto, the flexible radiation-protective sheet 12.

In **Fig. 1b** the first flap portion 32 has been raised from the first lowered position to the second raised position, here with it, and consequently the radiation-protective flap 30, substantially perpendicular to the flexible radiation-protective sheet 12. As seen, a first part of a of hook and loop attachment element 46 is now revealed on the side of the first flap portion 32 previously contacting the flexible radiation-protective sheet 12. Further, a second part of the hook and loop attachment element 48 is revealed on the second flap portion 34.

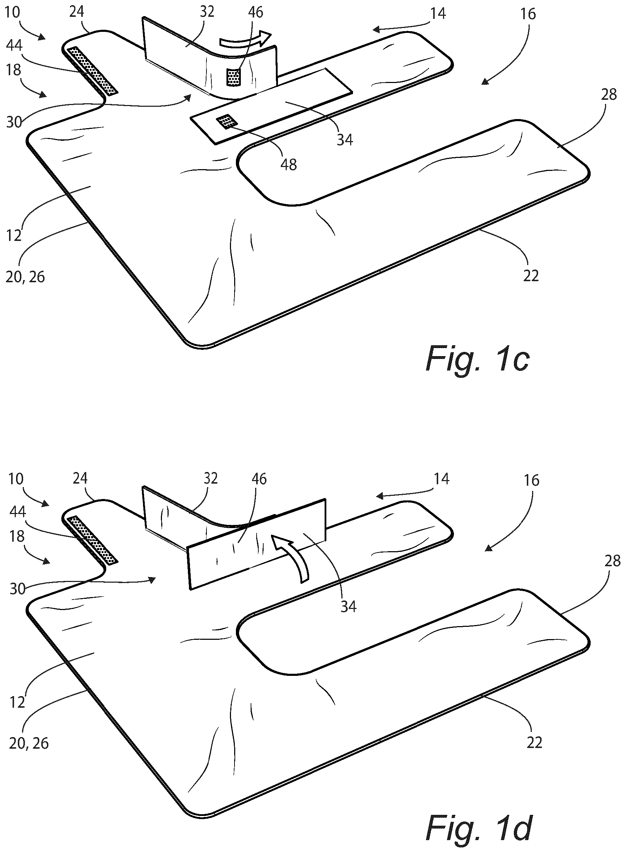

The first and second parts of the hook and loop attachment element 46, 48 constitute a retaining element for retaining the radiation-protective flap 30 in the second raised position, as will be revealed in full in Figs 1c to 1e.

If the radiation-protective flap 30 is implemented as only the first flap portion 32 or only the second flap portion 34, then a retaining element for retaining the retaining the radiation-protective flap 30 in the second raised position may for example be implemented by a shaping element as further shown in Fig. 1h, or by a stiff hook and loop attachment strip similar to hook and loop attachment strip 44, the stiff hook and loop attachment strip being attached to the flexible radiation-protective sheet 12 at one end and being attachable to the first part of hook and loop attachment element 44 at its second end.

**Fig. 1c** shows the first embodiment of the radiation-protective covering 10 with the first flap portion 32 curved to conform to a part of the border 20, in particular to a part of the border or circumference of the first peripheral cutout 14 in the flexible radiation-protective sheet 12.

As seen, by having the first flap portion 32 only be attached to the flexible radiation-protective sheet 12 over part of its length, as shown by arrow 36 in Fig. 1a, it is possible to both fold the first flap portion 32 from the first lowered position to the second raised position without difficulty, also facilitated by the attachment line 36 being substantially straight. Further, the non-attached part of the first flap portion 32 may then be bent or curved so as to better conform to the border or circumference of the first peripheral cutout 14.

Subsequently, as shown in **Fig. 1d**, the second flap portion 34 is folded to the second raised position so as to abut the first flap portion 32. As seen, the bent or curved part of the first flap portion 32 now abuts, and is supported and held in place by, the second flap portion 34

To finalize and retain the radiation-protective flap 30 in the second raised position, and as shown in **Fig. 1e**, the non-attached parts of the second flap portion 34, i.e. the part of the second flap portion which is not attached to the flexible radiation-protective sheet 12, is curved or bent to conform it to the first flap portion 32. This entails bringing the first and second parts of hook and loop attachment element 46, 48 in contact so as to attach the first and second flap portions 32, 34 to each other. In particular, as understandable from Fig. 1b and 1e, the first and second parts of hook and loop attachment element 46, 48 are provided on the non-attached parts of first and second flap portions 32, 34.

In the position shown in Fig. 1e, due to the attachment of the first and second flap portions 32, 34 by means of the first and second parts of hook and loop attachment element 46, 48, a stable structure has been formed and the radiation-protective flap 30 is retained in the second raised position. This is due to the first and second intersecting attachment lines 36, 38 which, due to being intersecting, i.e. not parallel but forming an angle between them if extended to intersect, cause any further folding of the first and second flap portions 32, 34, whether backwards towards the first lowered position, or forwards towards the first peripheral cutout 14, to result in movement between the first and second flap portions 32, 34, which movement is prevented by the attachment of the first and second flap portions 32, 34 to each other by means of the first and second parts of hook and loop attachment element 46, 48.

The flexible radiation-protective material of the flexible radiation-protective sheet 12 and the radiation-protective flap portions 32, 34, is a permeable fabric that comprises monofilaments arranged in a regular pattern and extending in a uniform direction, for example as described in WO2014163574A1. Each filament is solid composition that comprises a carrier substance of polyolefin and radiopaque substance of Antimony and Bismuth powder that are uniformly mixed. The fabric is a woven fabric with the filaments forming the weft of the cloth and with a warp of polyester. The filaments may extend along the width dimension of the flap portions 32, 34 to provide stiffness and allow curving of the flap portions in the second raised position. The filaments may extend in the length dimension of the flexible radiation-protective sheet 12, i.e. from the lower edge 22 towards the upper edge 24, to allow the flexible radiation-protective sheet 12 to better conform to the shape of the patient as seen in Fig. 2. The flexible radiation-protective material can attenuate X-ray radiation from a radiation source having a tube voltage of between 40 to 150 kV. The flexible radiation-protective material used in the embodiments of the radiation-protective covering shown in the figures has at least 0.50 mm Pb equivalence at any tube voltage in the range 60-110 kV.

**Fig. 1f** shows a second embodiment 10' of the radiation-protective covering. The second embodiment, i.e. radiation-protective covering 10', is identical to the first embodiment, i.e. radiation-protective covering 10, except for further comprising a radiation-protective patch 50 comprising a flexible radiation-protective material being releasably attached to the flexible radiation-protective sheet 12 via the hook and loop attachment strip 44 and using a hook and loop attachment strip 52 provided on the radiation-protective patch 50. The inclusion of the radiation-protective patch 50 is advantageous in that it allows to fully cover one or more peripheral cutout or fenestration, in Fig. 1f the third peripheral cutout 18, to protect from radiation when access to the patient through that peripheral cutout or fenestration is not needed. The flexible radiation-protective sheet 12 may be provided with hook and loop attachment strips, similar to hook and loop attachment strip 44, at numerous locations, such as at each peripheral cutout or fenestration. The flexible radiation-protective material of the radiation-protective patch 50 is the same as the flexible radiation-protective material of the flexible radiation-protective sheet 12 described above.

**Fig. 1g** shows a third embodiment of the radiation-protective covering. The third embodiment, i.e. radiation-protective covering 10", is identical to the first embodiment, i.e. radiation-protective covering 10, except for comprising two radiation-protective flaps 30, 30₁, each comprising first and second flap portions 32, 32₁ and 34, 34₁. Accordingly, in the radiation-protective covering 10" also the second peripheral cutout 16 is provided with a radiation-protective flap 30₁ for providing protection from radiation emanating or scattered from or through the second peripheral cutout 16.

**Fig. 1h** shows a fourth embodiment of the radiation-protective covering. The fourth embodiment, i.e. radiation-protective covering 10‴, is identical to the first embodiment, i.e. radiation-protective covering 10, except for the radiation-protective flap 30', in particular the modified first and second flap portions 32', 34', being provided with a shaping element 54, 54₁ and the second peripheral cutout 16 being replaced by a fenestration 16'.

Shaping element 54, 54₁ is implemented as a plastically deformable metal rod or wire that is integrated in the first and second flap portions 32', 34' along their outer edges as shown in more detail in Fig. 1i. As can be understood from Fig. 1h and Fig. 1e, the shaping elements 54, 54₁ can serve both as a retaining element for retaining the radiation-protective flap 30' in the second raised position by retaining the curved shape of the first and second flap portions 32', 34' in the position shown in Fig. 1e. As such, the first and second parts of the hook and loop attachment element 46, 48 may be dispensed with in the radiation-protective covering 10‴. Further, if the radiation-protective flap 30' is implemented as a single first or second flap portion, then the shaping element 54, by its capability of retaining a curved shape of the radiation-protective flap 30' in the second raised position, also functions as a retaining element for retaining the radiation-protective flap 30 in the second raised position.

Further, the shaping element 54, 54₁ can provide additional support to the outer edge, i.e. the upper edge in the second raised position, of the first and second flap portions 32', 34' in the second raised position. This may prevent the upper edge from sagging at positions far from the part of the first and second flap portions 32', 34' where the first and second flap portions 32', 34' overlap and/or are curved. Additionally, the shaping element 54, 54₁ allows the first and second flap portions 32', 34', and thus the radiation-protective flap 30' to be further shaped, e.g. by clinical staff, to even better conform to part of the border, e.g. to any curvature of the border of the peripheral cutout or fenestration and/or to even better protect against radiation emanating from or through the peripheral cutout or fenestration.

As noted above **Fig. 1i**, which shows an enlarged partial cross section of the fourth embodiment of the radiation-protective covering, further shows the placement of the shaping element 54 within and along an outer edge of the radiation-protective flap, in particular within and along an outer edge of the first flap portion 32'. Fig. 1i further shows one way of attaching the first flap portion 32', i.e. via stitches 56. As further seen, the first flap portion 32', and also the second flap portion 34', may be formed by two layers of flexible radiation-protective material folded such that the shaping element 54 may be placed in the fold at the upper edge.

**Fig. 2** shows a patient 4 laying on an operating table 2 and being partially covered by the radiation-protective covering 10, and further shows a C-arm imaging machine 6 and a clinical staff member 8. As seen in the enlargement in the lower part of Fig. 2, primary radiation 58 from the radiation source, below the operating table 2, of the C-arm medical imaging machine 6 passes through the operating table 2 and through the patient 4 through the first peripheral cutout 14. When the primary radiation 58 passes through the body of the patient 4, scattered radiation 60 forms and emanates through the first peripheral cutout 14. At least a part of the scattered radiation 60 is prevented from reaching the clinical staff member 8 by the radiation-protective flap 30, comprising the first and second flap portions 32, 34, which is in the second raised position.

As seen from Fig. 2, the radiation-protective covering 10 may typically have a length, from the lower edge 22 to the upper edge 24, of 20 to 100 cm, preferably 40 to 80 cm, and a width, from the left edge 26 to the right edge 28, of 20 to 100 cm, preferably 40 to 80 cm. Further, as can be appreciated from Fig. 2, the radiation-protective flap 30, if implemented as a single flap portions 32, may further be configured to have third position in which the radiation-protective flap 30 and the and second flap portions 32, 34 covers at least part of the first peripheral cutout 14.

### Item list

2 operating table
4 patient
6 C-arm medical imaging machine
8 clinical staff
10 radiation-protective covering
12 flexible radiation-protective sheet
14 first peripheral cutout
16 second peripheral cutout
18 third peripheral cutout
20 periphery
22 lower edge
24 upper edge
26 left edge
28 right edge
30 radiation-protective flap
32 first flap portion
34 second flap portion
36 arrow, attachment line
38 arrow, attachment line
40 arrow indicating width, or height, of flap portion
42 arrow indicating length of flap portion
44 hook and loop attachment strip
46 first part of hook and loop attachment element
48 second part of hook and loop attachment element
50 radiation-protective patch
52 hook and loop attachment strip
54 shaping element
56 stitching
58 primary radiation
60 scattered radiation

## Claims

1. A radiation-protective covering (10) comprising:
- a flexible radiation-protective sheet (12) comprising a flexible radiation-protective material, the flexible radiation-protective sheet having a border (20),
- a radiation-protective flap (30) comprising a flexible radiation-protective material, the radiation-protective flap being attached to the flexible radiation-protective sheet (12) adjacent a part of the border (20),
∘ wherein the radiation-protective flap (30) is configured to be foldable from a first lowered position in which the radiation-protective flap (30) is substantially parallel to the flexible radiation-protective sheet (12) to a second raised position.

2. The radiation-protective covering (10) according to claim 1, wherein the flexible radiation-protective sheet (20) comprises at least one fenestration (16') or peripheral cutout (14, 16, 18), and the part of the border is a part of a border of the at least one fenestration (16') or peripheral cutout (14, 16, 18).

3. The radiation-protective covering (10) according to any preceding claim, further comprising:
- a retaining element (46, 48) for retaining the radiation-protective flap (30) in the second raised position.

4. The radiation-protective covering (10) according to any preceding claim, wherein the radiation-protective flap has a length (42) in the plane of the flexible radiation-protective sheet (12) in the first lowered position, and wherein the radiation-protective flap (30) is attached to the flexible radiation-protective sheet along a part (36, 38) of its length (42).

5. The radiation-protective covering (10) according to any preceding claim, wherein the radiation-protective flap (30) comprises a shaping element (54) for retaining a shape of the radiation-protective flap (30) in the second raised position, preferably wherein the shaping element is arranged along a longitudinal edge of the radiation-protective flap, more preferably wherein the shaping element is arranged along a first longitudinal edge opposite to a second longitudinal edge along which the radiation-protective flap is attached to the flexible radiation-protective sheet (12).

6. The radiation-protective covering (10) according to any preceding claim, wherein the radiation-protective flap (30) comprises:
∘ a first flap portion (32) attached to the flexible radiation-protective sheet (12) adjacent a first part of the part of the border (20), and
∘ a second flap portion (34) attached to the flexible radiation-protective sheet (12) adjacent a second part, different from the first part, of the part of the border (20),
∘ and wherein the first and second flap portions (32, 34) overlap at least partially in the second raised position.

7. The radiation-protective covering (30) according to claim 6, wherein the first and second flap portions (32, 34) are attached to the flexible radiation-protective sheet (21) along corresponding first and second intersecting attachment lines (36, 38) in the plane of the flexible radiation-protective sheet (12), and
wherein the retaining element (46, 48) comprises attachment elements provided on the first and second flap portions (32, 34) for connecting the first and second flap portions to each other in the second raised position.

8. The radiation-protective covering (30) according to claim 7, wherein the first and second flap portions (32, 34) overlap with parts of the first and second flap portions that are not attached to the flexible radiation-protective sheet (12).

9. The radiation-protective covering (10) according to any of the claims 7-8, wherein the attachment elements (46, 48) are provided on parts of the first and second flap portions (32, 34) which overlap with each other.

10. The radiation-protective covering (10) according to any of the claims 7-9, wherein the attachment elements (46, 48) comprise one or more selected from the group consisting of hook and loop structures, magnets, snap fasteners, button and buttonhole, hook and eye, toggle and loop, twist lock, and buckles.

11. The radiation-protective covering (10) according to any of the preceding claims, wherein the radiation-protective flap (30) is attached to the flexible radiation-protective sheet by one or more of stitches, hook and loop, magnets, rivets, snap fasteners, welding, and adhesive.

12. The radiation-protective covering (30) according to any of the preceding claims, wherein the flexible radiation-protective sheet (20) comprises at least one fenestration (16') or peripheral cutout (14, 16, 18), the radiation-protective covering (30) further comprising:
- a radiation-protective patch (50) comprising a flexible radiation-protective material, the radiation-protective patch being configured to be attachable to the flexible radiation-protective sheet for at least partially covering the at least one fenestration [16') or peripheral cutout (14, 16, 18) in the flexible radiation-protective sheet (12).

13. The radiation-protective covering (30) according to any of the preceding claims, comprising at least two of at least one fenestration (16') or at least one peripheral cutout (14, 16, 18), and further comprising at least two radiation-protective flaps (30, 30₁).

14. The radiation-protective covering (10) according to any preceding claim, wherein the flexible radiation-protective material comprises:
a permeable fabric that comprises filaments, each filament being of a solid composition that comprises a carrier substance and a radiopaque substance, and the fabric is a woven fabric with the filaments forming the weft of the fabric, or
an impermeable sheet formed from a solid composition comprising a carrier substance and a radiopaque substance.

15. Use of the radiation-protective covering (30) according to any of the preceding claims, for protecting clinical staff (8) from radiation (60) during a medical intervention comprising the use of radiation.
